# EUROPEAN PATENT SPECIFICATION

(11) **EP 3 033 958 B1**
(45) Date of publication and mention of the grant of the patent: **04.01.2023**
(21) Application number: 15200246.5
(22) Date of filing: 15.12.2015
(51) Int. Cl.: A61B 5/00, A61B 5/0205, A43B 3/34, A61B 5/11

(54) **METHOD OF DETECTING A FALL OF A FOOTWEAR USER**
METHODE ZUR ERKENNUNG EINES STURZES EINES SCHUHBENUTZERS
MÉTHODE DE DÉTECTION DE CHUTE D'UN UTILISATEUR DE CHAUSSURES

(30) Priority: 19.12.2014 US 201414577509
(43) Date of publication of application: 22.06.2016
(73) Proprietor: Withings, 92130 Issy-les-Moulineaux (FR)
(72) Inventor: BESNARD, Marc, 75006 Paris (FR); OUIN, Jean-Raphaël, 92130 Issy les Moulineaux (FR); HUTCHINGS, Cédric, 92130 Issy les Moulineaux (FR); BUARD, Nadine, 92190 Meudon (FR)
(74) Representative: Withings IP

(56) References cited:
- WO-A2-2008/061023
- US-A- 5 678 448
- US-A1- 2007 260 421
- US-A1- 2009 038 182
- US-A1- 2012 123 277
- US-A1- 2013 176 126
- US-A1- 2013 213 145

## Description

### FIELD OF THE INVENTION

The invention relates to the field of wireless connected footwear and of associated detection methods detecting preferably one or more health and/or comfort parameters related to the footwear user or wearer.

### BACKGROUND OF THE INVENTION

It is known to use footwear to detect or to assess one or more health parameters related to the user or the wearer of this footwear. Therefore, this footwear often includes one or more sensors adapted to detect or to assess one or more health parameters related to the footwear user.

According to some prior art, for example described in American patents US 5449002, US 5608599, US 5662123, or in American patent US 5471405, or in American patents US 5323650, US 5678448, or in American patent US 6356856, or in American patent application US 2001046860, or in American patent US 6546813, or in international patent application WO02093272, or in American patent US 7713173, or in American patents US 7714711, US 7714709, US 7724132, US 7796027, or in American patent application US 2014135591, it is known to detect or to assess one or more health parameters related to the user or the wearer of a pair of shoes. Almost wherever the shoe wearer is, the relevant health parameters related to her or him may be detected or assessed, since the shoe wearer wears her or his shoes most of the time, in various life situations.

However, life situations where a shoe wearer acts are so different from one another that it may not be that easy to detect or assess sensed parameters in a consistent way, whatever the life situation, especially when this life situation is not known in advance by the processor monitoring the sensed parameters.

Moreover, since a shoe wearer has numerous different pairs of shoes, integrating in all of them the required system, that is the required sensor(s) or processor may become very expensive. On the contrary, if only a very limited number of pairs of shoes are equipped with the required sensor(s) or processor, then the relevant health parameters related to the shoe wearer may not be detected or assessed most of the time.

For health related metrics measurement, it is much more efficient to be able to make measurements in a controlled and constant environment as well as under controlled and constant conditions. For example, if one wants to compare a measurement to another measurement made the day or the week before, it is more efficient and more relevant to make such a comparison in the exactly same conditions or at least in very similar conditions. Slippers, being used at home, are part of this "helpful routine" allowing for more relevant comparisons and therefore are advantageous compared to shoes.

US5678448A is about a system for continuously measuring forces applied by the foot.

US2007/260421A1 is about athletic or other performance sensing systems.

US2009/038182 is about a footwear with built-in scale.

WO2008/061023A2 is about an electronic video system which incorporates the foot movements of a user into a video program.

US2013/213145A1 is about a footwear having a sensor system.

US2013/176126A1 is about a system for monitoring running steps.

US2012/123277A1 is about a method for detecting an extraordinary situation.

### SUMMARY OF THE INVENTION

The invention is defined in the appended claims.

One object of some embodiments of the present disclosure is specific footwear which will be worn by the user in more homogenous life situations, thereby allowing for a more consistent detection and assertion of relevant health parameters related to this footwear user.

Another object of some embodiments of the present disclosure is specific footwear which will be worn often, if not always, by the user in these more homogenous life situations, thereby allowing for a longer period of consistent detection and assertion of relevant health parameters related to this footwear user.

One or both of preceding objects may be fulfilled by providing a wireless connected indoors slipper comprising at least a sensor of at least a health or comfort parameter of said slipper user.

Another object of some embodiments of the present disclosure is to allow for detection or assertion of more health parameters, or for detection or assertion of other health parameters, or for more accurate detection or assertion of health parameters, or for easier detection or assertion of health parameters.

This object may be fulfilled by providing wireless connected footwear comprising at least a sensor which is a piezoresistive sensor or pneumatic sensor or an impedance sensor or an optic sensor or an acceleration sensor or a weight sensor or a pressure sensor.

Still another object of some embodiments of the present disclosure is to allow for detection or assertion of more health parameters, or for detection or assertion of other health parameters, thereby offering more complete or richer life situation monitoring. This object of course also includes a partial or total combination of sensors listed above.

This object may be fulfilled by providing a wireless connected footwear comprising at least a sensor of at least a health or comfort parameter of said footwear user, said health or comfort parameter being slipper user attitude or slipper user balance or slipper user heart rate or slipper user blood oxygenation or slipper user blood composition or slipper user hydration or slipper user pulse transit time or slipper user blood pressure or slipper user stroke volume or slipper user weight or slipper user temperature. This object of course also includes a partial or total combination of health or comfort parameters listed above. Still another object of some embodiments of the present disclosure is to improve footwear user fall risk detection.

This object may be fulfilled by providing a method of detecting a fall of a slipper user, wherein both a pressure exerted on a sole of said slipper and an acceleration of said slipper are detected by one or more sensors located in an indoors slipper and combined together to detect whether said user is fallen or not. Detecting whether said user is fallen or not will often amount to detect a level of fall risk for said user, since it may be difficult to know with certainty whether said user is indeed fallen or not.

Still another object of some embodiments of the present disclosure is to alleviate at least partly the above mentioned prior art drawbacks.

Preferred embodiments comprise one or more of the following features, which can be taken separately or together, either in partial combination or in full combination, with any of preceding objects of the disclosure. Preceding objects of the disclosure may be combined among each other too.

According to an embodiment of the disclosure, the wireless connected indoors slipper also comprises a microprocessor adapted to perform a pretreatment on said sensed health or comfort parameter(s) so as to provide information or an alert representative of health or of comfort of said slipper user. Performing a sufficient pretreatment within the slipper allows for needing to send less information outside this slipper, while keeping a good balance between effectiveness of the system, which is sufficient useful information provided, and false alarm rate, which amounts to avoiding as much as possible providing useless information.

According to an embodiment of the disclosure, the wireless connected indoors slipper also comprises an emitter adapted to wirelessly send a signal representative of said information or of said alert. The emitter allows for sending out of the slipper some of the useful information that will treated thereafter. Wireless communication makes it more comfortable for the slipper user.

According to an embodiment of the disclosure, said emitter is adapted to wirelessly send said signal to a cell telecommunication device. Thereby, the useful information may be used in an application located on the cell telecommunication device, which may be a cell phone or a smart phone, and the result given by this application either displayed on the cell telecommunication device screen or sent to another electronic device where another person than the slipper user may be alerted if necessary.

According to an embodiment of the disclosure, said wireless signal is a Bluetooth signal or a Bluetooth Low Energy signal. This short range protocol is perfectly adapted to such short range communication, while requiring low energy so requesting only a small battery integrated within the slipper. Other suitable short range protocols may be used alternatively. For example, WIFI or Zigbee may be used instead of Bluetooth.

According to an embodiment of the disclosure, said sensor is a piezoresistive sensor or a pneumatic sensor or an impedance sensor or an optic sensor or an acceleration sensor. This embodiment of course also includes a partial or total combination of sensors listed above.

According to an embodiment of the disclosure, said health or comfort parameter is slipper user attitude or slipper user balance or slipper user heart rate or slipper user blood oxygenation or slipper user blood composition or slipper user hydration or slipper user pulse transit time or slipper user blood pressure or slipper user stroke volume or slipper user weight or slipper user temperature. This embodiment of course also includes a partial or total combination of health or comfort parameters listed above.

According to an embodiment of the disclosure, said sensed health or comfort parameter(s) is or are representative of a behavior of said slipper user. Thereby, the behavior of the slipper user or at least part of it may be reconstructed a posteriori, merely by detecting or assessing some health or comfort parameters related to this slipper user.

According to an embodiment of the disclosure, said sensor is integrated in a sole of said slipper. A substantive proportion of the evaluated parameters will be more or less dependent on the pressure or on the pressure variations exerted by user foot on slipper and more particularly on slipper sole.

According to an embodiment of the disclosure, said sensor is sandwiched between two superposed layers of said sole. This complete embedding of sensor within slipper sole will better protect this sensor from any external aggression.

According to an embodiment of the disclosure, said sole is detachable of said slipper. Thereby, the sole with its equipment may be kept by the slipper user, when this slipper user chooses to change her or his worn slippers.

According to an embodiment of the disclosure, said sole is flexible, to the contrary of shoes whose sole is rigid.

According to an embodiment of the disclosure, sole thickness ranges between 4 mm and 12mm and preferably from 5 to 10 mm.

According to an embodiment of the disclosure, said sensor or one of said sensors is a weight or pressure sensor adapted to detect a force or a pressure exerted on a sole of said slipper. Pressure or pressure variations are one of the most interesting detectable parameters related to health and comfort of slipper user.

According to an embodiment of the disclosure, said pressure sensor extends over more than a half of said sole of said slipper. Then pressure or pressure variations may be more accurately detected or assessed making the system globally more effective.

According to an embodiment of the disclosure, both detected pressure and detected acceleration are wirelessly sent out of said slipper.

According to an embodiment of the disclosure, a slipper user fall risk alert is sent when a sequence of a sudden sensed pressure drop shortly followed by a sudden sensed acceleration raise is detected and/or when a sudden sensed pressure drop is detected simultaneously with a sudden sensed acceleration raise. Shortly followed here means preferably a fraction of a second or one second, or may extend to a few seconds.

According to an embodiment of the disclosure, said fall risk alert is wirelessly sent out of said slipper. According to another embodiment of the disclosure, both detected pressure and detected acceleration are wirelessly sent out of said slipper towards a remote communication device, and said fall risk alert is computed in said remote communication device.

Simultaneous detection and assessment of pressure and of acceleration, as well as the relationship between pressure and acceleration changes, may indeed help system to more accurately detect and evaluate fall risk from the slipper user.

According to an embodiment of the disclosure, each time the slipper user puts on her or his slippers, there is an audio signal, like for example a "bip", showing that the slippers are ON and operational.

According to an embodiment of the disclosure, each time the slipper user puts her or his slippers on a battery charger, there is an audio signal, like for example a "bip", showing that the slippers are being charged.

Further features and advantages of the invention will appear from the following description of embodiments of the invention, given as non-limiting examples, with reference to the accompanying drawings listed hereunder

### BRIEF DESCRIPTION OF THE DRAWINGS

Fig. 1 shows an example of a situation of a user wearing indoors slippers according to an embodiment of the invention.
Fig. 2 shows an example of an indoors slipper according to an embodiment of the invention.
Fig. 3 shows an example of a method performing detection of a fall of user wearing indoors slippers according to an embodiment of the invention.

### DETAILED DESCRIPTION OF THE INVENTION

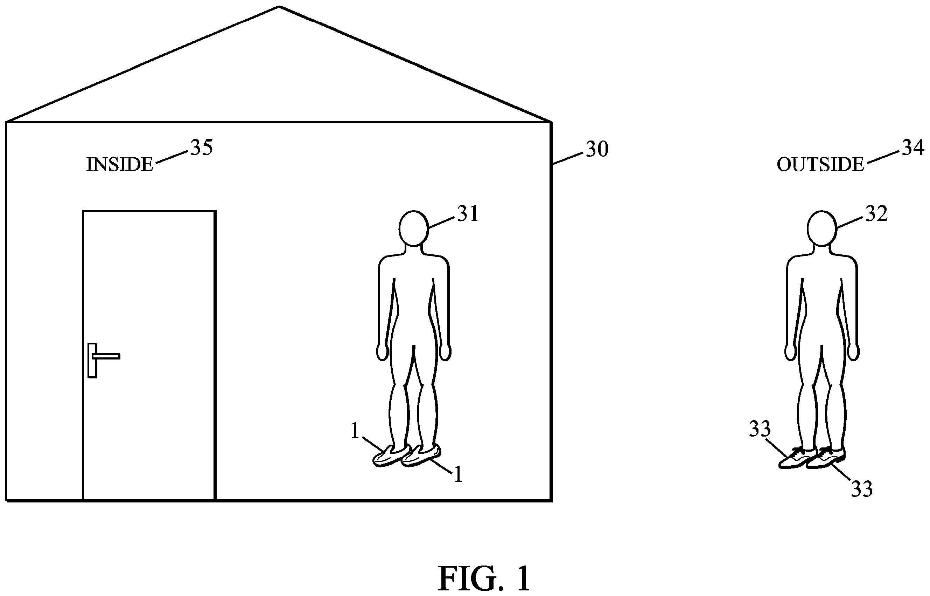

Fig. 1 shows an example of a situation of a user wearing indoors slippers according to an embodiment of the invention. There is a house 30. There is an inside 35 and an outside 34, both delimited with respect to each other by the house 30 itself. Any other type of building, block, apartment, or similar construction, instead of the house 30, would do too.

When being outside, a person 32 is wearing a pair of shoes 33 most of the time. The person 32 has many different pairs of shoes 33. Therefore, either a majority of those shoes 33 integrates the system according to embodiments of invention, or most of the time, the person 32 health or comfort will not be monitored. Life situations, like walking, running, jumping, swimming, riding, driving, diving are quite numerous and very different as far as the position and move of the body of the person 32 are concerned, as well as with respect to many health or comfort parameters related to this person 32. Therefore, without knowing in advance in which life situation the person 32 is, it may be more difficult for the system to differentiate between two similar parameter variations happening in two very different life situations.

When being inside, a person 31 is wearing a pair of slippers 1 most of the time. The person 31 has very few pairs of slippers 1, and usually a single pair of slippers 1. Therefore, it will quite easy to integrate the system in one or two different pairs of slippers 1. Thereby, health and comfort of the slipper user may be easily monitored most of the time. Life situations are not so different for a person 31, when being inside a known construction like a house 30. Indeed, most of the time, the person 31 will be walking, standing or sitting. Therefore, it will be much easier not to mix up and not to misinterpret health and comfort parameters related to the person 31. Besides, inside activities usually happen to be more regular, from one day to the next, than outside activities. Hence, again, inside health or comfort parameter monitoring will be easier and more accurate inside than outside.

Therefore, integrating this system monitoring health or comfort parameters related to a person wearing footwear, will be both more cost effective for the footwear and more secure with respect to detection and assessment of those health or comfort parameters, if this footwear is a slipper or a pair of slippers as proposed by the invention, rather than if this footwear is a shoe or a pair of shoes as described in the prior art of record.

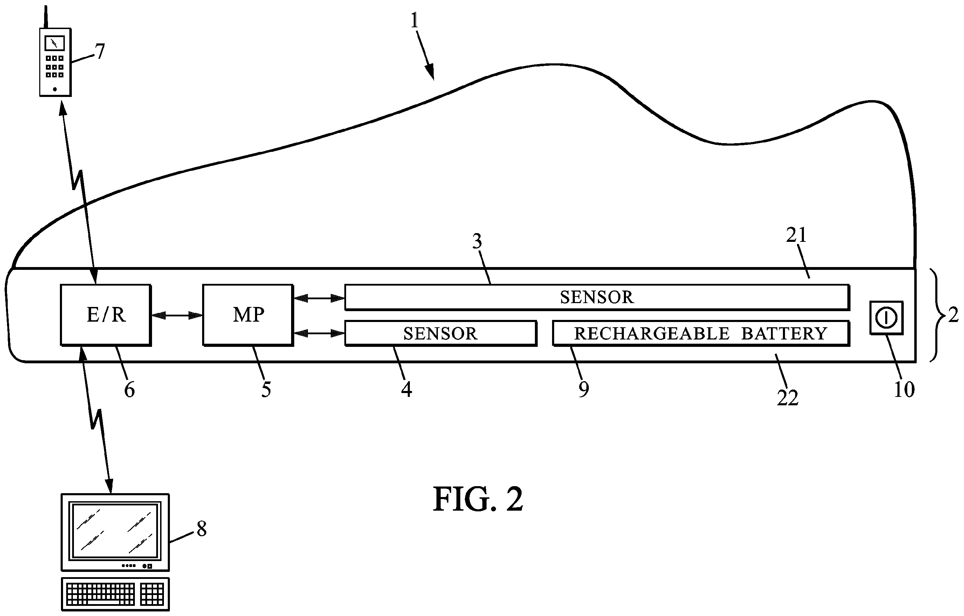

Fig. 2 shows an example of an indoors slipper according to an embodiment of the invention. One slipper only in a pair or both slippers of the pair may be equipped with the elements which will now be described in more detail in an embodiment of the invention. Some of these elements may also be functionally shared by both slippers but structurally disposed in only one of them. In this latter case, some communication between both slippers of a pair is introduced.

A slipper 1 has a sole 2. The sole 2 integrates a first sensor 3 and a second sensor 4. Both sensors 3 and 4 are sensing one or more health or comfort parameters related to the user of slipper 1 which is the wearer of slipper 1. Both sensors 3 and 4 will respectively communicate with a processor 5, either by wired communication or by wireless communication, so as to send sensed parameters to the processor 5. Preferably, sensors 3 and 4 will, frequently or even consistently, send sensed parameters to processor 5. On the contrary, processor 5 will perform some pretreatment or even some analysis on the received parameters so as to distinguish when and which information to forward to the transmitter 6.

The transmitter 6 may be an emitter / receiver 6 or a mere emitter 6. The transmitter 6 will wirelessly transmit pretreated information or an alert to a remote communication or telecommunication device for further treatment or analysis or monitoring. The remote device may be for example a cell telecommunication device 7, like a cell phone or a smartphone or a tablet or a connected watch adapted to wirelessly communicate with the slipper or a connected weight scale adapted to wirelessly communicate with the slipper, being able to call another telecommunication device or to send him a message. The remote device may also, be alternatively or comprise simultaneously, a personal computer 8, with internet link or not, for example being then able to send mails to other personal computers. The transmitter 6 may send a signal to the remote device 7 and/or 8 advantageously each few seconds, for example from one signal every second to one signal every ten seconds, the sent signal being periodical or not.

All elements integrated within the slipper 1, sensors 3 and 4, processor 5, transmitter 6, need to be powered. A power supply may be a rechargeable battery 9 which powers all elements integrated within the slipper 1. This rechargeable battery may be recharged either by a plug and socket system not shown on figure 2, or by a charging system without contact like a charging system operating on the principle of induction, no plug and socket assembly being needed in this latter case. Other types of power supply devices may be used, like for example non rechargeable but long lasting batteries, power supply device which can recharged by recovering the energy of the movement of the slipper user, or another type of suitable power supply. The sole 2 may advantageously integrate a switch 10 which switches the slipper 1 between ON state and OFF state at will. Slipper 1 user may switch it ON when he puts his slippers 1 and switch it OFF when he removes his slippers 1. This will avoid false alarm sending when the user doesn't wear his slippers 1, and hence reduce the false alarm rate. Switch 10 may alternatively automatically detect whether there is a user foot within the slipper 1 or not.

The sensors 3 and 4 may be sandwiched between layers of the sole 2. The sole 2 may have at least an upper layer 21 and a lower layer 22, as well as one or more intermediate layers. Sensor 3 may be a sensor which is a piezoresistive sensor or a pneumatic sensor or an impedance sensor or an optic sensor or an acceleration sensor or a weight sensor or a pressure sensor. Sensor 4 may be a sensor which is a piezoresistive sensor or a pneumatic sensor or an impedance sensor or an optic sensor or an acceleration sensor or a weight sensor or a pressure sensor. Sensors 3 and 4 may be both of the same type of sensor or alternatively may be of different types of sensor from each other. There also may be more than two sensors, either of same or different type, within the sole 2 of slipper 1. One or some or all sensors may also be in a part of the slipper 1 which is different from the sole 2.

Sensor 3 may sense a slipper user attitude or slipper user balance or slipper user heart rate or slipper user blood oxygenation or slipper user blood composition or slipper user hydration or slipper user pulse transit time or slipper user blood pressure or slipper user stroke volume or slipper user weight or slipper user temperature. Sensor 4 may sense a slipper user attitude or slipper user balance or slipper user heart rate or slipper user blood oxygenation or slipper user blood composition or slipper user hydration or slipper user pulse transit time or slipper user blood pressure or slipper user stroke volume or slipper user weight or slipper user temperature. Sensors 3 and 4 may both sense either the same type of parameter or alternatively may sense different types of parameters from each other. There also may be more than two sensed parameters, either of same or different type. A weight sensor of slipper may be calibrated by an electronic scale wirelessly communicating with the slipper, when the slipper user stands on the electronic scale.

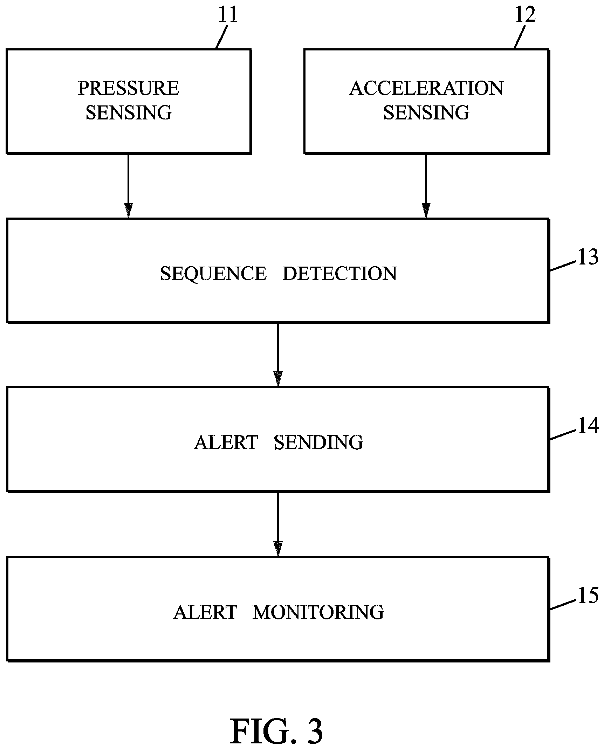

Fig. 3 shows an example of a method performing detection of a fall of user wearing indoors slippers according to an embodiment of the invention.

In a first step, both a pressure sensing 11 and an acceleration sensing 12 are performed.

The results of those pressure and acceleration values variations are then used in a second step of sequence detection 13. Both pressure and acceleration variations are monitored and specific relationships between pressure variations and acceleration variations are associated to corresponding slipper user fall risk levels. For instance, the detection of a sudden pressure drop quickly followed by a sudden acceleration raise will be associated to a high risk of slipper user fall risk.

Once a certain threshold of slipper user fall risk is exceeded, an alert is sent in a third step of alert sending 14. This alert may be sent to one or more remote devices. For instance, this alert may be sent to the smart phone of the slipper user, which in turn will phone a relative of this slipper user or a close hospital integrating this "at home watching" monitoring service subscribed by the slipper user. This alert may also be sent to a personal computer and stored in memory for being later retrieved and analyzed, so as to anticipate future fall risks of this slipper user by knowing as soon as possible some stumbling of this slipper user, before any fall really happens.

Once the alert sent, this alert will be monitored in a fourth step of alert monitoring 15. This alert, depending on the alert risk level and on the use configuration of the system, will be monitored either by medical staff when being worn in a hospital, or by a relative of the slipper user, or by a paid companion, or by a private nurse, or by whoever is in the house or close to the house and may react in a useful manner to help the possibly fallen slipper user.

This method of slipper user fall risk monitoring may be particularly well suited for elderly or frail persons. Even if such a product is not meant to replace medical monitoring by medical staff, this product may be a helpful device in some usual life situations for some categories of persons needing it.

The slipper can also be in wireless communication with a Home monitor. Such a home monitor is typically a stand-alone, connected device placed in a home room, configured to acquire pictures, videos; additionally, such home monitor is able to measure the ambient temperature of the room together with the humidity level; also the air quality is monitored by such home monitor; in particular, it can comprise a carbon dioxide concentration sensor, a Volatile Organic Compound (VOC) sensor, other gas or dust sensor. Picture/video shooting can be event driven, including triggered upon some unexpected air quality data.

The invention has been described with reference to preferred embodiments. However, many variations are possible within the scope of the invention determined by the appended claims.

## Claims

1. A method of detecting a fall of a footwear user wherein both a pressure exerted on a sole of said footwear and an acceleration of said footwear are detected (11, 12) by one or more sensors located in a footwear and combined together to detect whether said user is fallen or not,
wherein a footwear user fall risk alert (14) is sent when a sequence of a sudden sensed pressure drop shortly followed by a sudden sensed acceleration raise is detected and/or when a sudden sensed pressure drop is detected simultaneously with a sudden sensed acceleration raise, wherein said fall risk alert is wirelessly sent out of said footwear.

2. The method of claim 1, further comprising storing the fall risk alerts in a remote device for being later retrieved and analyzed, so as to anticipate future fall risks of the footwear user.

3. The method of any of claims 1-2, wherein the footwear is a slipper.

4. The method of any of claims 1-3, wherein "shortly" extends to a few seconds.

5. The method of any of claims 1-4, wherein "shortly" means a fraction of a second or one second.

## Patentansprüche

1. Verfahren zum Erkennen eines Sturzes eines Schuhwerkbenutzers, wobei sowohl ein auf eine Sohle des Schuhwerks ausgeübter Druck als auch eine Beschleunigung des Schuhwerks durch einen oder mehrere in einem Schuhwerk angeordnete Sensoren erfasst (11, 12) und miteinander kombiniert werden, um zu erkennen, ob der Benutzer gestürzt ist oder nicht, wobei ein eine Warnung über das Sturzrisiko des Schuhbenutzers (14) gesendet wird, wenn eine Sequenz auss einem plötzlichen erfassten Druckabfall kurz gefolgt von einem plötzlichen erfassten Beschleunigungsanstieg erfasst wird und/oder wenn ein plötzlicher erfasster Druckabfall gleichzeitig mit einem plötzlichen erfassten Beschleunigungsanstieg erfasst wird, wobei die Warnung über das Sturzrisiko des Schuhbenutzers drahtlos aus dem Schuhwerk gesendet wird.

2. Das Verfahren nach Anspruch 1 umfasst ferner das Speichern die Warnung über das Sturzrisiko des Schuhbenutzers in einer entfernten Vorrichtung, um sie später abzurufen und zu analysieren, so dass Sturzrisiken des Schuhbenutzers zu antizipieren.

3. Verfahren nach einem der Ansprüche 1-2, wobei das Schuhwerk ein Hausschuh ist.

4. Das Verfahren nach einem der Ansprüche 1 bis 3, wobei sich "kurz" auf einige Sekunden erstreckt.

5. Verfahren nach einem der Ansprüche 1 bis 4, wobei "kurz" einen Bruchteil einer Sekunde oder eine Sekunde bedeutet.

## Revendications

1. Procédé de détection d'une chute d'un utilisateur d'article chaussant dans lequel une pression exercée sur une semelle dudit article chaussant et une accélération dudit article chaussant sont détectées (11, 12) par un ou plusieurs capteurs situés dans un article chaussant et combinées ensemble pour détecter si ledit utilisateur est tombé ou non,
dans lequel une alerte de risque de chute de l'utilisateur de l'article chaussant (14) est envoyée lorsqu'une séquence d'une chute soudaine de pression captée suivie de peu par une augmentation soudaine d'accélération captée est détectée et/ou lorsqu'une chute soudaine de pression captée est détectée simultanément avec une augmentation soudaine d'accélération captée,
dans lequel ladite alerte de risque de chute est envoyée sans fil hors dudit article chaussant.

2. Le procédé de la revendication 1, comprenant en outre le stockage des alertes de risque de chute dans un dispositif distant pour être récupérées et analysées ultérieurement, de manière à anticiper les risques de chute futurs de l'utilisateur de l'article chaussant.

3. Le procédé de l'une quelconque des revendications 1-2, dans lequel l'article chaussant est un chausson.

4. Le procédé de l'une quelconque des revendications 1-3, dans lequel « de peu » s'étend à quelques secondes.

5. Procédé de l'une quelconque des revendications 1 à 4, dans lequel « de peu » signifie une fraction d'une seconde ou une seconde.
